# EUROPEAN PATENT SPECIFICATION

(11) **EP 1 379 330 B1**
(45) Date of publication and mention of the grant of the patent: **03.11.2004**
(21) Application number: 02722549.9
(22) Date of filing: 10.04.2002
(51) Int. Cl.: B01J 19/08, C02F 1/30

(54) **METHOD FOR PURIFYING AND REFINING FLUIDS THROUGH ACCELERATED ELECTRONS**
VERFAHREN ZUR REINIGUNG EINER FLÜSSIGKEIT MITTELS BESCHLEUNIGTEN ELEKTRONEN
PROCEDE POUR PURIFIER ET RAFFINER DES FLUIDES AU MOYEN D'ELECTRONS ACCELERES

(30) Priority: 13.04.2001 IT TO20010372
(43) Date of publication of application: 14.01.2004
(73) Proprietor: Perona, Silvio, 10139 Torino (IT)
(72) Inventor: Perona, Silvio, 10139 Torino (IT)
(74) Representative: Robba, Pierpaolo
(86) International application number: PCT/IB2002/001165
(87) International publication number: WO 2002/083295

(56) References cited:
- EP-A- 0 953 554
- WO-A-95/03374
- DE-A- 2 337 406
- DE-A- 2 546 756
- DE-A- 3 413 318
- DE-C- 19 740 401
- GB-A- 665 263
- US-A- 4 072 591
- US-A- 4 184 956
- US-A- 5 357 291
- US-A- 5 378 898
- US-A- 5 523 577

## Description

The present invention refers to a method utilising high-energy accelerated electrons, typically from 1.2 MeV to 1.8 MeV, for purifying, recovering, refining or chemically modifying fluid substances of different origins, particularly waste waters, oily sludges, exhausted oils, crude oil and natural gas, whose treatment is deemed to be necessary for the safeguard of the environment or for industrial applications.

For a long time now, laboratory trials have shown that beams of electrons having high kinetic energy directed towards a solid mass, or a liquid mass, cause inside said mass a series of cascade collisions with the atoms forming the substance, inducing their displacement and an outstanding ionisation together with a strong electromagnetic radiation.

Subsequent to said strong interactions, when the electrons are completely slowed down and an energetic re-balancing among the ions generated by the collisions has occurred, it is present inside the substance subject to the treatment, together with an overall heating, a variety of new chemical products in variable percentages due to the recombination reactions; the creation of synthesised products-subsequent to the electrons action does not simply affect a single layer of few fractions of µm below the surface of the irradiated bodies, such as in the ion implantation, but it also affects deeper layers.

However, in consequence of the lack of use of specific reaction chambers as well as of a convenient electrons flow, only in a few peculiar cases (such as the cross-linking in the polymers) an extensive and homogeneous production of new compounds inside large volumes subject to the treatment has been noticed, therefore accelerated electrons have not been used so far in the industrial field to purify, to refine or to synthesise new substances.

Generally the electronic beams apparatuses are employed in some sterilisation processes (foodstuff, packages or materials for medical use), or to harden the polymeric materials surface, or in all that cases where it is necessary to modify the "surface properties" of some industrial products, but they are not used to change "volume properties".

It is obvious, though, that a new treatment technology based on accelerated electrons, able to act on the whole mass of a substance as well as to modify its properties, without requesting a remarkable power supply, would be very interesting due to the deriving benefits, even with respect to the rapidity and simplicity of the process.

The scope of the method according to the present invention is, therefore, to conveniently combine the electronic emission with the configuration of the reaction chamber, and to obtain, with a relatively moderate increasing of the intensity of the electrons flux with respect to the standards valid for the surfaces treatment, an high accentuation of the dissociation activity and of the subsequent aggregation of the treated substances.

From this point of view, the present invention makes use of the electrons projection at high energy by employing very intense beams consisting of said negative charge particles, whose parameters are totally controlled, as well as it provides a shielded reaction chamber able to keep inside itself the electromagnetic radiations generated therein, as well as it controls the flow of the substance fed into said reaction chamber for the treatment, said substance having to be fluid in order to be exposed progressively and for the necessary time to the action of the electrons and of the relevant radiolysis.

Further to the above arrangements, which are imperative to achieve reactions affecting the whole mass subject to the treatment and concurrently assuring a moderate energy dispersion, this invention foresees to add vibratory elastic stresses and catalysts.

By performing the operations according to the above description, particularly by using a reaction chamber properly configured to promote the interaction between the mass therein contained and the electrons beam, the method according to the present invention is therefore suitable for the treatment of fluids (liquids or gases) of whichever origin and it can be advantageously employed in many industrial activities, particularly in those activities where waste products generated during the manufacturing processes have to be purified or recovered as well as where chemical, petrochemical or metallurgical basic products have to be refined or modified.

To achieve the purpose above mentioned, the subject of present invention refers to a method for purifying, recovering, refining or chemically modifying fluid substances of different origins, whose treatment is deemed to be necessary for the safeguard of the environment or for industrial applications, characterised in that it comprises the following operations:
a) to prepare the fluid within a suitable chamber, equipped with a shielding able to reflect the electromagnetic radiations inside of the mass of the substance to be treated and to direct towards said fluid a beam consisting of high-energy accelerated electrons;
b) to accurately control the fluid flow, the electrons flux (number of negative charges per unit of surface area and per unit of time) and the energy supplied to said electrons, by keeping said parameters within the peculiar values at which the desired reactions take place in the treated substance;
   and, moreover, characterised in that further to the electrons irradiation realised by means of a proper accelerator able to supply energy contents up to 1.8 MeV (more generally up to 12 MeV), one or both of the following additional operations are also provided:
c) to put catalysts in said reaction chamber, locating them at the edging positions of the region where the strong interactions among the electrons occur, said catalysts being apt to increase the desired reactions in the treated mass;
d) to furthermore include inside said reaction chamber some ultrasonic transducers, of piezoelectric or magnetostrictive type, and to activate with vibratory elastic waves the mass subject to the treatment.

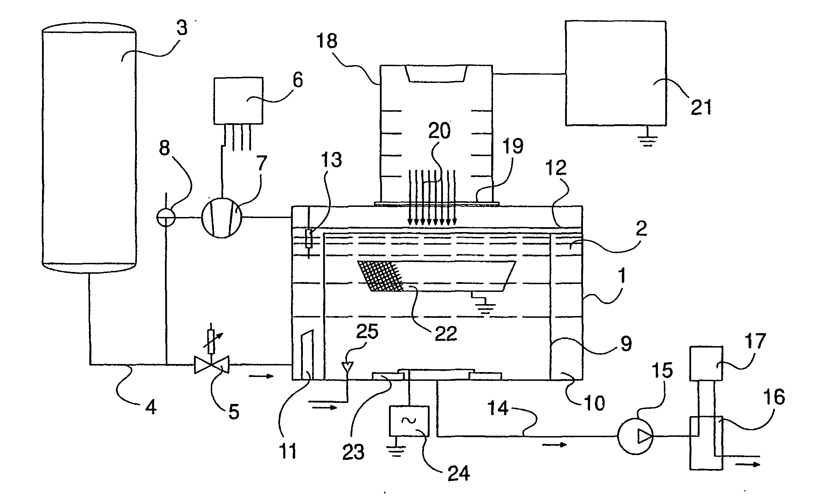

The method according to the present invention will be hereinafter described in detail with reference to the accompanying drawing, enclosed hereto for purposes of not limiting illustration, wherein Figure 1 is the schematic representation of a continuous-flow apparatus suitable to perform the method in accordance with this invention.

With reference to Figure 1, the numeral 1 qualifies the chamber used to contain the fluid 2 to be subject to the treatment, a liquid in the specific case, previously stocked inside the tank 3. Through the duct 4, the liquid 2 is fed into the chamber 1, with a flow regulated by means of the electrovalve 5; said valve is a proportional valve, i.e. it is able to choke the liquid flow rate depending on the electric signals transmitted by the control unit 6, which is in charge of the process control.

Inside of chamber 1, the pressure is maintained at a value lower than the atmospheric pressure value by means of the vacuum pump 7; depending on the fact that the sucked substance is air (starting phase) or vapours released from the liquid, they are open discharged through the valve 8 or re-fed (compressed) into the duct 4. Due to the low pressure values set inside the chamber 1, with the exception of a very viscous liquid 2, it is not necessary to provide an injection pump to continuously feed said chamber 1.

The vacuum pump 7, like any other device described hereinafter, is controlled by the control unit 6 connected thereto by means of shielded electric cables, or fibre optic cables, not represented in the scheme.

Inside the chamber 1, a second chamber, qualified with the numeral 9, having a cylinder tank shape and being cover-less in its upper part is provided, within which the fluid 2 overflows when the cavity 10, which divides said two chambers, is completely filled up; while ascending inside of said cavity 10, said liquid 2 gets a light rotational movement due to a baffle 11 placed in front of the injection point of the duct 4.

With the exclusion of the starting phase when the liquid 2 to be subject to the treatment starts to be fed, the chamber 1 and the hold-up second internal chamber 9 are always filled up with said liquid 2; more particularly said liquid 2 fills up the volume portion delimited from the second internal chamber 9 because the liquid level, qualified with the numeral 12, is regularly maintained higher than its superior edge. The liquid level is controlled by means of the measuring unit 13, which is located aside of the second internal chamber 9, said measuring unit 13, connected to the control unit 6, supplying to said control unit 6 the signals to operate the progressive opening or closing of the electrovalve 5.

The liquid 2 accumulated in the upper part of the second internal chamber 9 (which is actually the real reaction chamber) is subject to the electrons bombardment until it descends towards the lower part of the same, and this happens due to the suction performed by the pump 15, located on the duct 14 communicating with the bottom. In short, the liquid 2 fed into the chamber 1, goes up along the cavity 10 and goes down inside the second internal chamber 9, where is treated, afterwards it is brought outside.

Down the pump 15, there is the slot 16 wherein the probes 17 measure the physico-chemical characteristics of the treated liquid and they send their signals to the control unit 6, for the possible convenient variations of the equipment operating parameters.

Over the chamber 1 is placed the linear accelerator, qualified in the scheme with the numeral 18, which, through a diaphragm 19, conveys the electrons beam 20 towards the central region of the second internal chamber 9 filled up with the liquid 2, which continuously flows down. The accelerator 18 is supplied by the high-voltage unit 21, and it can also be a non-linear accelerator depending on what better fits the equipment configuration (cyclotron, betatron, resonant cavities).

The depression generated inside the chamber 1 by the suction pump 7, said depression being also only of some tenth of bar, has the scope of reducing the energy dispersion of the electrons directed towards the liquid 2 and, above all, of minimising the pressure difference with respect to the accelerator 18, wherein high-vacuum conditions are maintained; in this way, the diaphragm 19 can have a reduced thickness or the flow area of the electronic beam can be increased. Said diaphragm 19 can also consist of a single crystal having atomic layers oriented so to duct the electrons and to improve the coherent electromagnetic radiation (i.e., an electromagnetic radiation wherein defined wavelengths prevail); alternatively the single crystal foil can be located in a different place.

The energy level up to which the electrons have to be accelerated to achieve a good yield of the method under subject changes a lot depending on the type of the treated liquid, and according to this variety the accelerators are "tailor-made" with respect to each equipment, nevertheless enabling a fine tuning of the values of the above mentioned energy level. In most applications, said energy is comprised between 1.2 MeV and 1.8 MeV, but it can also be much lower without contrary directions.

On the contrary, the energy superior threshold is conditioned by the presence of Deuterium or Beryllium inside the substance to be treated. These elements may release significant percentages of neutrons when the threshold of 1.8 MeV is exceeded, and it is imperative to make sure that no substantial traces are present before proceeding with higher energies; in said conditions it is possible to operate even at 12 MeV, but this it is not essential to perform the present invention.

The powers to be supplied by means of the accelerator 18 may considerably change too depending on the dimensions of the equipment and, above all, on the kind of the treated liquid. Average parameters, for the purification of water, are for instance 1.2 MeV as far as the value of energy supplied to each electron is concerned, and 10¹⁶ electrons per litre of substance (~2KGy); referring to an equipment which treats 72 m³/hour, the above average parameters correspond to a supply of direct current of 32 mA, with a power of 39 kW. Different values, even appreciably higher, are requested to accomplish the refining of hydrocarbons or the synthesis of other substances.

The electrons beam 20 that hits from above the liquid 2 accumulated in the reaction chamber, as already described hereinbefore, is progressively slowed down while encountering the layers of dense matter and in the same way the deceleration radiation (bremsstrahlung) or the radiation of interaction with the atoms is absorbed, therefore at a certain distance from the level 12 at which said liquid 2 is continuously maintained, the direct action of the electrons disappears. However, the last reactions do not take place where the strongest collisions occur but in the region directly underlying, i.e. where the disequilibrium phase among the treated substance components has been mostly spread and where the radiolysis is still present.

The liquid regular downwards flow, performed by means of the suction pump 15, merely follows, therefore, the reactions accomplishment as well as it brings the liquid already treated to the second internal chamber 9 bottom; as a general rule, when half said second internal chamber 9 has been overcome, the recombination reactions already occurred and the liquid is then in its stable and definitive phase.

In case the treatment providing the sole electronic irradiation does not completely attain the desired targets, in terms of yield or quality of the obtained product, the method according to the present invention enables to strengthen new compounds formation by means of a catalyst, qualified with the numeral 22 in Figure 1; said catalyst, very approximately represented, is placed in the central region of second internal chamber 9, at the edging positions of the region where the electrons interact in the strongest way.

The catalyst 22 is shaped in the form of a wire mesh made basket, but the shape and the shaping material can considerably change depending on the treated substances; the catalyst can be made of foils or spongy masses, a pure metal or an alloy can be used, nevertheless always taking care of the fact that its location must not directly interfere with the electronic beam. The catalyst 22 location in the middle zone of the second internal chamber 9 is also useful to quickly ground, without generation of detrimental electric fields, the electric charges accumulating in the liquid 2 while the electronic irradiation treatment occurs.

A further chance to increase and to correctly direct the formation of useful substances following the energization generated by the electrons irradiation is to activate the mass contained in the second internal chamber 9 with ultrasonic waves. To this purpose, on the bottom of said second internal chamber 9 transducers 23 supplied by means of a high-frequency generator 24 are located.

Said transducers of piezoelectric type, or alternatively of magnetostrictive type, which are oscillating due to the synchronised electric signal transmitted by the generator 24, transmit to the liquid contained into the second internal chamber 9 vibratory elastic waves, whose scope is to promote its stirring as well as its stabilisation; the powers transmitted by means of said devices are not to be necessarily high, being sufficient for the main part of the applications the generation of a light action in the liquid under treatment, without causing cavitation phenomena.

The use of ultrasonic apparatuses requires a proper isolation, nevertheless chamber 1 itself is conveniently realised with particular arrangements sufficient to avoid the ultrasonic waves diffusion in the surroundings.

As a matter of fact the metallic internal walls of chamber 1 must be glossed with particular electrochemical treatments so to show a surface adapted to reflect towards the liquid 2 the electromagnetic emissions of various wavelengths. Since, however, a part of the radiation is anyway absorbed and reemitted, said chamber 1 is coated with appropriate linings made of composites to avoid its diffusion in the surroundings; this kind of cover layered or woven, is able to absorb the ultrasonic waves too.

The apparatuses now described allow to perform the method according to the present invention, wherein a liquid fed into a reaction chamber is modified through the electrons irradiation; the synergetic use of catalysts and ultrasounds together with the electrons action allows to increase and improve in terms of effectiveness the reactions.

Said apparatuses configuration can also be changed, for instance the liquid flow can be inverted or it can be fed into the equipment from second internal chamber 9 bottom, changing therefore the location of the pump 15 with the valve 5 together with the relevant ducts of feeding and discharge; consequently the catalyst 22 configuration is modified as well as its position, which has to be closer to the superior edge of second internal chamber 9.

By performing the above different way of feeding, the liquid movement is directed towards the electronic beam 20 and the stabilisation of the substances produced during the treatment takes place when the liquid overflows into the cavity 10; in substance, the effectiveness of the purification or refining process does not change, but it can be more convenient to adopt the above different feeding flow in case of very viscous liquids or when pulverised solids are dispersed therein.

Even a significant density variation between the substance fed into the equipment and the substance discharged after treatment makes preferable the above kind of feeding, i.e. the feeding of the liquid 2 from the second internal chamber 9 bottom, to avoid re-mixings between the heaviest phase still to be treated, which is inclined to fall downwards, with the lightest phase already treated.

On the contrary, in case a sole gaseous fluid has to be treated whatever way of feeding can be chosen; in this case, the gas or the vapours can be injected under pressure into chamber 1, so giving up the advantage of using a very thin diaphragm 19. In the contrary case that the gases have to be mixed with a liquid phase to react, it is certainly convenient to inject them from the second internal chamber 9 bottom and to disperse them in the overhead liquid 2 by means of appropriate diffusers 25. The product deriving from the treatment, a mixture of liquid compounds produced during the reaction with the gas, could be discharged through the bottom of the second internal chamber 9 or of the cavity 10 depending on the feeding flow direction given to the liquid 2.

A great variety of substances can be subject to the method according to the present invention with surprisingly good results, particularly with respect to the low energy consumption due to the reaction chamber equipped to hold inside itself and to reflect the electromagnetic radiations; many waste liquids, or gases, generated from industrial productions or civil uses, contain pollutants or infectious agents, which can be totally or partially neutralised by employing the electronic irradiation. In this case the electrons action mainly generates, as final effect, the polluting metals oxidation or the cracking of detrimental substances complex molecules and it enables the discharge to the environment of the fluids previously containing said pollutants.

The same process as above can be applied to oily sludges and to exhausted oils with respect to which the electronic irradiation treatment, co-operating with the catalysts and the ultrasounds, produces alterations such to allow their re-use, even under a different form (essentially as fuels). As a matter of fact, when treating hydrocarbons polymerised molecules, the reactions more easily induced by the high-energy electronic beam involve the chain bonds, which are cracked due to said reactions, therefore producing mixtures of products having lower molecular weights (gas oils, gasolines). In addition to mineral oils, even several plastics are suitable to be recovered: objects or manufacturing operations residual products made of polyethylene, polypropylene, polystyrene and synthetic rubber, previously ground and dispersed within the mass of liquid hydrocarbons to be refined, are modified themselves into valuable fuels through the same cracking phenomenon of their macromolecules polymeric bonds.

Even more advantageously the present invention can be applied to the crude oil refining. Said mineral can be subject to the method according to the invention without preliminary refining modifications through the fractional distillation, even in case of a very dense mixture originated from shales and bituminous sands; by using a molybdenum-based catalyst 22 also the desulfurization is more easily achieved.

The presence of a very various hydrocarbons mixture in the crude oil does not limit the efficiency of the present method, which acts in the direction to produce final products having a chain shorter than the initial ones; it behaves, in substance, as the cracking does, without being bound to a preliminary selection of the different compounds. This characteristics allows, then, to adopt the method under subject also in sites different from the traditional refinery, for instance directly nearby the wells that extract the crude oil from the reservoirs.

The possibility to directly operate on the extraction site allows, what is more, to recover the gaseous fractions that spontaneously split off from the crude oil, even without achieving a real refining of said crude oil; very often said gases, which are mixtures of variable percentages of methane, ethane, propane, etc., are burned at mouth of well or pumped into the ground because their transport is not convenient, but the irradiation with electrons of said gases mixed to the crude oil produces liquid hydrocarbons of mean molecular weight, more valuable and even more easily transportable within oil pipelines with respect to the crude oil originally extracted. The low molecular weight paraffinic gases, in fact, due to their relative abundance of hydrogen atoms can bind themselves with fragments of more complex molecules, particularly of unsaturated hydrocarbons, and form liquid products globally lighter.

The crude oil so "enriched" has to be then subject to a further method of electronic irradiation, or of traditional refining, to obtain the final modification into products for industrial use.

As already said hereinabove, the method under subject substitutes the cracking without the necessity of preliminarily splitting the crude oil into its components having different evaporating temperatures, however with respect to some kinds of crude oil containing significant percentages of valuable products, the fractional distillation can be realised at the beginning and the sole remaining less valuable fraction can be subject to the electronic irradiation treatment in order to improve its quality.

Also the hydrocarbons homogeneous mixtures, already deriving from a fractionation, can be treated with the electrons irradiation to obtain their structural alteration and their transformation into isoparaffin waxes (increase of the contained isomers percentage); in these cases the catalyst 22 function proves to be particularly emphasised, said catalyst being chosen of the suitable material (generally iron, nickel or cobalt), which contributes to the recombination of the molecular fragments into branched chains instead of linear chains.

Same utility is provided by the present invention with respect to the natural gas treatment, mainly methane, which can be advantageously used for producing liquid fuels.

It is non dealt, in this case, with the recover in remote regions of otherwise wasted resources but with a further possibility of employing the methane abundantly available in many industrial areas.

Said gas can be utilised to increase the gas oils and gasolines production by adopting the same device used for recovering at crude oil wells, where it is released in minimal quantities; in this case, on the contrary, inside the refinery it is possible to split the most favourable crude oil liquid fraction in terms of producing the desired fuels, and to feed it into the equipment for the electronic irradiation together with the gas in well define rates.

Without further devices, this particular kind of alkylation process, obtained by means of the method according to the present invention, enables to increase the production of automotive fuels of about 30-50%, by converting an equal mass of natural gas.

In case of complete lack of the crude oil, the present invention allows, nevertheless, the treatment of water and natural gas (or pulverised carbon) in accordance to the reforming method and without involving the high-temperature vapour production; following the same route it is possible to produce valuable fuels starting from vegetal oils or from other biomasses mixed with water.

Among the liquid substances that can be treated according to the present invention the molten glazing or the molten metals, which have to be purified and alloyed, are to be considered; the action realised by the electronic irradiation, in fact, influences both the processes to obtain the oxides reduction and the way in which the metals combine themselves to form an alloy, and it enables mixtures otherwise unrealisable through the sole solubility of the various components.

The metals treatment does not require a different equipment from the one described in Figure 1, except for the fact that the chamber 9 practically becomes a ladle containing the molten metal and said metal is poured therein directly at the superior edge; the process can be a continuous-flow process with the treated metal discharge from the bottom of chamber 9 through a drain-trap that pours the metal into ingot moulds or into atomisers for a rapid solidification.

In case the continuous-flow treatment is not employable, the molten metal can be progressively poured into chamber 9 replacing a melting pot. By letting the metal solidify inside said melting pot during the treatment unusual alloys can be more easily obtained, said alloys having better mechanical characteristics.

The potential applications of the method according to the present invention are, after all, numberless being possible to treat whatever kind of fluid, liquid or gas, only requiring the identification, case by case, of the most convenient operative parameters: configuration of the reaction chamber, electrons energy level, emission intensity, choice of a precise catalyst and/or activation of ultrasonic waves, fluid flow rate and so on.

Obviously, standing the invention principle, the details of construction, the forms of actuation and the ways of application could be largely changed with respect to the description herein referred without, for this reasons, falling out from the scope of the present invention.

## Claims

1. A method for purifying, recovering, refining or chemically modifying liquid fluids of different types by electronic irradiation treatment, comprising the steps of:
a) preparing a fluid-tight reaction chamber (1) having metallic internal walls;
b) feeding a fluid (2) into said chamber (1);
c) irradiating said fluid (2) into said chamber (1) with a high-energy accelerated electrons beam (20) in order to improve its quality;
d) removing said fluid (2) from said reaction chamber (1),
**characterised in that** inside said reaction chamber (1), a second internal chamber (9) divided from said reaction chamber (1) by a cavity (10) and cover-less in its upper part is provided, and **in that** means (14, 15) are provided for maintaining the level (12) inside said reaction chamber (1) higher than the superior edge of said second internal chamber (9) while causing said fluid (2) to overflow from said cavity (10) into said second internal chamber (9) or viceversa, whereby said fluid (2) is homogeneously subjected to the electrons bombardment and to the electromagnetic radiations generated inside said chambers (1, 9) during its continuous and regular downward flow until it reaches the lower part of said second internal chamber (9) or of said cavity (10).

2. A method according to claim 1, **characterised in that** said electronic beam (20) owns an energy of less than 12 MeV, said energy being preferably comprised between 1.2 MeV and 1.8 MeV.

3. A method according to claim 1, **characterised in that**
- means are provided able to accurately control the flow of said fluid (2),
- means are provided able to accurately control the intensity of said electronic beam (20) (said intensity referred to as the number of negative charges per unit of surface area and per unit of time),
- means are provided able to accurately control the electrons energy level of said beam (20),
- means are provided with the aim of increasing the reaction rates inside the fluid (2),
- means are provided with the aim of inhibiting the accumulation of excess electric charges and the generation of outstanding electric fields inside said fluid (2),
- means are provided with the aim of promoting the stirring as well as the stabilisation of said fluid (2).

4. An equipment for purifying, recovering, refining or chemically modifying liquid fluids of different types by electronic irradiation treatment, comprising:
a) a fluid-tight reaction chamber (1) having metallic internal walls;
b) a tank (3) containing a fluid (2);
c) means (4, 5) to transfer said fluid (2) from said tank (3) into said chamber (1);
d) means (18) placed over said chamber (1) to irradiate said fluid (2) into said chamber (1) with a high-energy accelerated electrons beam (20) in order to improve its quality;
e) means (14, 15) to remove said fluid (2) from said chamber (1),
**characterised in that** inside said reaction chamber (1), a second internal chamber (9) divided from said reaction chamber (1) by a cavity (10) and cover-less in its upper part is provided, and **in that** means (14, 15) are provided for maintaining the level (12) inside said reaction chamber (1) higher than the superior edge of said second internal chamber (9) while causing said fluid (2) to overflow from said cavity (10) into said second internal chamber (9) or viceversa, whereby said fluid (2) is homogeneously subjected to the electrons bombardment and to the electromagnetic radiations generated inside said chambers (1, 9) during its continuous and regular downward flow until it reaches the lower part of said second internal chamber (9) or of said cavity (10).

5. An equipment according to claim 4, **characterised in that** said means (14, 15) comprises a suction pump (15) located on a duct (14) communicating with the bottom of said second internal chamber (9) or with the bottom of said cavity (10).

6. An equipment according to claim 4, **characterised in that** said means (18) to irradiate said fluid (2) are represented by an electron accelerator able to emit an high-energy electrons beam (20) placed over the fluid (2) surface and directed towards said surface, and by a diaphragm (19), or an alternative target, consisting of a single crystal having atomic layers oriented exactly along the path of said beam (20) interposed between said accelerator (18) and said fluid (2) surface.

7. An equipment according to claims 4, **characterised in that** inside said reaction chamber (1) as well as inside said second internal chamber (9) a pressure is maintained lower than the atmospheric pressure.

8. An equipment according to claims 4 and 7, **characterised in that** said fluid (2) is fed into said reaction chamber (1) through said cavity (10) and it is continuously and regularly upwardly moved inside said cavity (10) due to the pressure gradient between the atmospheric pressure and the pressure inside said chamber (1).

9. An equipment according to claims 4 and 7, **characterised in that** said fluid (2) is fed into said reaction chamber (1) through said second internal chamber (9) and it is continuously and regularly upwardly moved inside said second internal chamber (9) due to the pressure gradient between the atmospheric pressure and the pressure inside said chamber (1).

10. An equipment according to claim 4, **characterised in that** the internal surface of said reaction chamber (1) as well as of said second internal chamber (9) is glossed or coated with a reflecting material so to improve the electromagnetic radiations reflection.

11. An equipment according to claim 4, **characterised in that** it includes, inside said chamber (1), one or more metallic catalysts (22) suitable for fostering the reaction rates within the treated fluid (2) and for grounding the excess electric charges, said metallic catalysts (22) being located at the edging positions of the region defined in said reaction chamber (1), where said electrons and said fluid (2) interact in the strongest way.

12. An equipment according to claim 4, **characterised in that** it includes, inside said chamber (1), one or more ultrasonic transducers (23), of piezoelectric or magnetostrictive type, able to activate the treated fluid (2) with vibratory elastic waves, in order to promote the stirring and the stabilisation of said fluid (2).

## Patentansprüche

1. Verfahren zum Reinigen, Regenerieren, Raffinieren oder chemischen Modifizieren von flüssigen Fluids verschiedener Art durch elektronische Bestrahlungsbehandlung, die folgende Verfahrensschritte aufweist:
a) Bereitstellung einer flüssigkeitsdichten Reaktionskammer (1) mit metallischen Innenwänden;
b) Zuführung von Fluid (2) in jene Kammer (1);
c) Bestrahlung jenes Fluids (2) in jener Kammer (1) mit einem hochenergetisch beschleunigten Elektronenstrahl (20) zu seiner Qualitätsverbesserung;
d) Entfernung jenes Fluids (2) aus der Reaktionskammer (1),
**dadurch gekennzeichnet, daß** innerhalb der Reaktionskammer (1) eine zweite, von jener Reaktionskammer (1) durch eine Aussparung (10) getrennte und in ihrem oberen Teil nicht abgedeckte zweite innere Kammer (9) vorgesehen ist und daß Einrichtungen (14, 15) zur Aufrechterhaltung eines Niveaus (12) innerhalb der Reaktionskammer (1) vorgesehen sind, das oberhalb des obersten Randes jener zweiten inneren Kammer (9) liegt und dabei jenes Fluid (2) veranlasst, von jener Aussparung (10) in jene zweite innere Kammer (9) oder umgekehrt überzufließen, wobei jenes Fluid (2) während seiner kontinuierlichen und regelmäßigen Abwärtsströmung bis zum Erreichen des unteren Teils der zweiten Innenkammer (9) oder der Aussparung (10) dem gleichmäßigen Elektronenbeschuss und der innerhalb jener Kammern (1, 9) erzeugten elektromagnetischen Strahlung ausgesetzt ist.

2. Verfahren nach Anspruch 1,
**dadurch gekennzeichnet, daß** der Elektronenstrahl (20) eine Energie von weniger als 12 MeV besitzt und die Energie vorzugsweise zwischen 1,2 und 1,8 MeV liegt.

3. Verfahren nach Anspruch 1,
**dadurch gekennzeichnet, daß**
- Einrichtungen vorgesehen sind, die geeignet sind, die Strömung des Fluids (2) genau zu steuern,
- Einrichtungen vorgesehen sind, die geeignet sind, die Stärke des Elektronenstrahls (20) genau zu steuern (die Stärke wird auf die An zahl der Negativladungen pro Oberflächeneinheit und pro Zeiteinheit bezogen),
- Einrichtungen vorgesehen sind, die geeignet sind, das elektronische Energieniveau des Elektronenstrahls (20) genau zu steuern,
- Einrichtungen mit der Zielsetzung der Steigerung der Reaktionsgeschwindigkeit innerhalb des Fluids (2) vorgesehen sind,
- Einrichtungen mit der Zielsetzung der Unterbindung der Anhäufung von überschüssigen elektrischen Ladungen und der Bildung von außergewöhnlichen elektrischen Feldern innerhalb des Fluids (2) vorgesehen sind,
- Einrichtungen mit der Zielsetzung, sowohl die Umwälzung als auch die Beruhigung des Fluids (2) zu fördern.

4. Eine Ausrüstung zum Reinigen, Regenerieren, Raffinieren oder chemischen Modifizieren von flüssigen Fluids verschiedener Art durch elektronische Bestrahlungsbehandlung, die Folgendes aufweist:
a) eine flüssigkeitsdichte Reaktionskammer (1) mit metallischen Innen wänden;
b) einen ein Fluid (2) enthaltenden Behälter (3);
c) Einrichtungen (4, 5) zur Förderung des Fluids (2) von jenem Behälter (3) in jene Kammer (1);
d) über jener Kammer (1) angeordnete Einrichtungen (18) zur Bestrah lung des Fluids (2) in der Kammer (1) mit einem zur Verbesserung seiner Qualität hochenergetisch beschleunigten Elektronenstrahl (20);
e) Einrichtungen (14, 15), um das Fluid (2) aus jener Kammer (1 ) zu entfernen,
**dadurch gekennzeichnet, daß** innerhalb der Reaktionskammer (1) eine zweite, von jener Reaktionskammer (1) durch eine Aussparung (10) getrennte und in ihrem oberen Teil nicht abgedeckte zweite innere Kammer (9) vorgesehen ist und daß Einrichtungen (14, 15) zur Aufrechterhaltung eines Niveaus (12) innerhalb der Reaktionskammer (1) vorgesehen sind, das oberhalb des oberen Randes jener zweiten inneren Kammer (9) liegt und dabei jenes Fluid (2) veranlaßt, von jener Aussparung (10) in jene zweite innere Kammer (9) oder umgekehrt überzufließen, wobei jenes Fluid (2) während seiner kontinuierlichen und regelmäßigen Abwärtsströmung bis zum Erreichen des unteren Teils der zweiten Innenkammer (9) oder der Aussparung (10) dem gleichmäßigen Elektronenbeschuß und der innerhalb jener Kammern (1, 9) erzeugten elektromagnetischen Strahlung ausgesetzt ist.

5. Ausrüstung nach Anspruch 4,
**dadurch gekennzeichnet, daß** jene Einrichtungen (14, 15) eine Saugpumpe (15) aufweisen, die in einer Leitung (14) angeordnet ist, die mit dem Boden der zweiten inneren Kammer (9) oder mit dem Boden der Aussparung (10) verbunden ist.

6. Ausrüstung nach Anspruch 4,
**dadurch gekennzeichnet, daß** jene Einrichtungen (18) zur Bestrahlung jenes Fluids (2) durch einen Elektronenbeschleuniger verkörpert werden, der geeignet ist, einen hochenergetischen Elektronenstrahl (20) zu emittieren, der über der Oberfläche des Fluids (2) angeordnet und gegen jene Oberfläche gerichtet ist, sowie durch ein Diaphragma (19) oder ein alternatives Ziel, das aus einem Einkristall besteht, dessen Atomlagen genau entlang der Bahn des Strahls (20) ausgerichtet sind und das zwischen dem Beschleuniger (18) und der Oberfläche des Fluids (2) zwischengeschaltet ist.

7. Ausrüstung nach Anspruch 4,
**dadurch gekennzeichnet, daß** sowohl innerhalb der Reaktionskammer (1) als auch innerhalb der zweiten Kammer (9) ein niedrigerer Druck als Atmosphärendruck aufrechterhalten wird.

8. Ausrüstung nach den Ansprüchen 4 und 7,
**dadurch gekennzeichnet, daß** jenes Fluid (2) durch die Aussparung (10) in die Reaktionskammer (1) gefördert wird und infolge des Druckgradienten zwischen dem Atmosphärendruck und dem Druck innerhalb der Kammer (1) kontinuierlich und regelmäßig innerhalb der Aussparung (10) aufwärts bewegt wird.

9. Ausrüstung nach den Ansprüchen 4 und 7,
**dadurch gekennzeichnet, daß** jenes Fluid (2) durch die zweite innere Kammer (9) in die Reaktionskammer (1) gefördert wird und infolge des Druckgradienten zwischen dem Atmosphärendruck und dem Druck innerhalb der Kammer (1) kontinuierlich und regelmäßig innerhalb der zweiten inneren Kammer (9) aufwärts bewegt wird.

10. Ausrüstung nach Anspruch 4,
**dadurch gekennzeichnet, daß** die Innenfläche sowohl der Reaktionskammer (1) als auch der zweiten inneren Kammer (9) poliert oder mit einem reflektierenden Material beschichtet ist, um so die Reflexion der elektromagnetischen Bestrahlung zu verbessern.

11. Ausrüstung nach Anspruch 4,
**dadurch gekennzeichnet, daß** sie innerhalb der Kammer (1) einen oder mehrere metallische Katalysatoren (22) beinhaltet, die zur Förderung der Reaktionsgeschwindigkeiten und zur Nullung überschüssiger elektrischer Ladungen innerhalb des behandelten Fluids zweckmäßig sind, wobei jene metallischen Katalysatoren (22) in den Randbereichen jener in der Reaktionskammer (1) definierten Regionen angeordnet sind, wo die Elektronen und jenes Fluid (2) am stärksten aufeinander einwirken.

12. Ausrüstung nach Anspruch 4,
**dadurch gekennzeichnet, daß** sie innerhalb der Kammer (1) einen oder mehrere Ultraschallwandler (23) der piezoelektrischen oder magnetostriktiven Art enthält, die in der Lage sind, das behandelte Fluid (2) mit elastischen Wellenschwingungen zu aktivieren, um die Umwälzung und die Stabilisierung des Fluids (2) zu fördern.

## Revendications

1. Procédé pour purifier, récupérer, raffiner ou modifier chimiquement des fluides liquides de différents types par traitement d'irradiation aux électrons, comprenant les étapes consistant à :
a) préparer une chambre de réaction étanche au fluide (1) comportant des parois métalliques internes ;
b) fournir un fluide (2) dans ladite chambre (1) ;
c) irradier ledit fluide (2) dans ladite chambre (1) avec un faisceau d'électrons accélérés sous haute énergie (20) afin d'améliorer sa qualité ;
d) retirer ledit fluide (2) de ladite chambre de réaction (1),
**caractérisé en ce qu'**est prévue, à l'intérieur de ladite chambre de réaction (1), une seconde chambre interne (9) séparée de ladite chambre de réaction (1) par une cavité (10) et découverte sans sa partie supérieure , et **en ce que** des moyens (14, 15) sont prévus pour maintenir le niveau (12) à l'intérieur de ladite chambre de réaction (1) plus haut que le bord supérieur de ladite seconde chambre de réaction interne (9) tout en entraînant ledit fluide (2) à déborder de ladite cavité (10) dans ladite seconde chambre interne (9) ou vice-versa, de façon que ledit fluide (2) soit soumis de façon homogène au bombardement d'électrons et aux rayonnements électromagnétiques générés à l'intérieur desdites chambres (1, 9) pendant son écoulement continu et régulier vers le bas jusqu'à ce qu'il atteigne la partie inférieure de ladite seconde chambre interne (9) ou de ladite cavité (10).

2. Procédé selon la revendication 1, **caractérisé en ce que** ledit faisceau électronique (20) possède une énergie inférieure à 12 MeV, ladite énergie étant, de préférence, comprise entre 1,2 MeV et 1,8 MeV.

3. Procédé selon la revendication 1, **caractérisé en ce que**
- des moyens sont fournis, capables de contrôler l'écoulement dudit fluide (2),
- des moyens sont fournis, capables de contrôler de façon précise l'intensité dudit faisceau électronique (20) (ladite intensité étant rapportée comme étant le nombre de charges négatives par unité de surface et par unité de temps),
- des moyens sont fournis, capables de contrôler de façon précise le niveau d'énergie des électrons dudit faisceau (20),
- des moyens sont fournis dans le but d'augmenter les vitesses de réaction à l'intérieur du fluide (2),
- des moyens sont fournis dans le but d'empêcher l'accumulation de charges électriques en excès et la génération de champs électriques hors normes à l'intérieur dudit fluide (2),
- des moyens sont fournis dans le but de favoriser l'agitation de même que la stabilisation dudit fluide (2).

4. Dispositif pour purifier, récupérer, raffiner ou modifier chimiquement des fluides liquides de différents types par traitement d'irradiation aux électrons, comprenant :
a) une chambre de réaction étanche aux fluides (1) comportant des parois métalliques internes ;
b) un réservoir (3) contenant un fluide (2) ;
c) des moyens (4, 5) pour transférer ledit fluide (2) dudit réservoir (3) dans ladite chambre (1) ;
d) des moyens (18) placés sur ladite chambre (1) pour irradier ledit fluide (2) dans ladite chambre (1) avec un faisceau d'électrons accélérés sous haute énergie (20) afin d'améliorer sa qualité ;
e) des moyens (14, 15) pour extraire ledit fluide (2) de ladite chambre (1),
**caractérisé en ce qu'**est fournie, à l'intérieur de ladite chambre de réaction (1), une seconde chambre interne (9) séparée de ladite chambre de réaction (1) par une cavité (10) et découverte dans sa partie supérieure, et **en ce que** des moyens (14, 15) sont fournis pour maintenir le niveau (12) à l'intérieur de ladite chambre de réaction (1) plus haut que le bord supérieur de ladite seconde chambre interne (9) tout en entraînant ledit fluide (2) à déborder de ladite cavité (10) dans ladite seconde chambre interne (9) ou vice-versa, de façon que ledit fluide (2) soit soumis de façon homogène au bombardement électronique et aux rayonnements électromagnétiques générés à l'intérieur desdites chambres (1, 9) pendant son écoulement continu et régulier vers le bas jusqu'à ce qu'il atteigne la partie inférieure de ladite seconde chambre interne (9) ou de ladite cavité (10).

5. Dispositif selon la revendication 4, **caractérisé en ce que** lesdits moyens (14, 15) comportent une pompe d'aspiration (15) placée sur un conduit (14) communiquant avec le bas de ladite seconde chambre interne (9) ou avec le bas de ladite cavité (10).

6. Dispositif selon la revendication 4, **caractérisé en ce que** lesdits moyens (18) pour irradier ledit fluide (2) sont représentés par un accélérateur d'électrons capable d'émettre un faisceau d'électrons de haute énergie (20) placé sur la surface du fluide (2) et dirigé vers ladite surface, et par un diaphragme (19), ou une autre cible, constituée d'un monocristal présentant des couches atomiques orientées exactement le long de la trajectoire dudit faisceau (20) interposé entre ledit accélérateur (18) et la surface dudit fluide (2).

7. Dispositif selon la revendication 4, **caractérisé en ce que**, à l'intérieur de ladite chambre de réaction (1) de même qu'à l'intérieur de ladite seconde chambre interne (9), une pression est maintenue inférieure à la pression atmosphérique.

8. Dispositif selon les revendications 4 et 7, **caractérisé en ce que** ledit fluide (2) est fourni à ladite chambre de réaction (1) par ladite cavité (10) et qu'il se déplace de façon continue et régulière vers le haut à l'intérieur de ladite cavité (10) en raison du gradient de pression entre la pression atmosphérique et la pression à l'intérieur de ladite chambre (1).

9. Dispositif selon les revendications 4 et 7, **caractérisé en ce que** ledit fluide (2) est amené dans la chambre de réaction (1) à travers ladite seconde chambre interne (9) et qu'il se déplace de façon continue et régulière vers le haut à l'intérieur de ladite seconde chambre interne (9) en raison du gradient de pression entre la pression atmosphérique et la pression à l'intérieur de ladite chambre (1).

10. Dispositif selon la revendication 4, **caractérisé en ce que** la surface interne de ladite chambre de réaction (1), de même que celle de ladite seconde chambre interne (9) est polie ou revêtue d'un matériau réfléchissant de façon à améliorer la réflexion du rayonnement électromagnétique.

11. Dispositif selon la revendication 4, **caractérisé en ce qu'**il comprend, à l'intérieur de ladite chambre (1), un ou plusieurs catalyseur(s) métallique (s) (22) approprié(s) pour favoriser les vitesses de réaction à l'intérieur du fluide traité (2) et pour neutraliser les charges électriques en excès, lesdits catalyseurs métalliques (22) étant placés au niveau des positions de bordure de la zone définie dans ladite chambre de réaction (1), où lesdits électrons et ledit fluide (2) interagissent de façon la plus forte.

12. Dispositif selon la revendication 4, **caractérisé en ce qu'**il comprend, à l'intérieur de ladite chambre (1), un ou plusieurs transducteur (s) ultrasonique (s) (23), de type piézoélectrique ou magnétostrictif, capables d'activer le fluide traité (2) avec des ondes élastiques vibratoires, afin de favoriser l'agitation et la stabilisation dudit fluide (2).
